# EUROPEAN PATENT APPLICATION

(11) **EP 4 745 976 A1**
(43) Date of publication of application: **20.05.2026**
(21) Application number: 24839283.9
(22) Date of filing: 07.05.2024
(51) Int. Cl.: G16H 10/00

(54) **PROGRAM, SPECIFIC EVENT RELATED DISPLAY DEVICE, SPECIFIC EVENT RELATED DISPLAY METHOD, AND RECORDING MEDIUM**

(30) Priority: 13.07.2023 JP 2023115108
(71) Applicant: NEC Solution Innovators, Ltd., Koto-ku, Tokyo 136-8627 (JP)
(72) Inventor: KOU, Yukari, Tokyo 136-8627 (JP); TANAKA, Hirofumi, Tokyo 136-8627 (JP); TAMURA, Natsuko, Tokyo 136-8627 (JP)
(74) Representative: Betten & Resch
(86) International application number: PCT/JP2024/016978
(87) International publication number: WO 2025/013385

(57) **Abstract**

Provided is a program realizing display that can increase individual awareness of health management, including an information acquisition procedure, a display screen information generation procedure, and an information output procedure, which are executed by a computer. The information acquisition procedure acquires future prediction information of a subject, including future physical and mental state prediction information, and future living condition prediction information, of the subject. The future living condition prediction information of the subject includes information on prediction of living condition associated with event occurrence, and information on prediction of living condition associated with absence of event occurrence. The display screen information generation procedure generates a display screen including information on occurrence risk of the specific event, the information on prediction of living condition associated with event occurrence, and the information on prediction of living condition associated with absence of event occurrence. The information output procedure outputs the display screen.

## Description

### INCORPORATION BY REFERENCE

This application is based upon and claims the benefit of priority from Japanese patent application No. 2023-115108, filed on July 13, 2023, the disclosure of which is incorporated herein in its entirety by reference.

### TECHNICAL FIELD

The present invention relates to a program, a specific event related display apparatus, a specific event related display method and a recording medium.

### BACKGROUND ART

As a tool for grasping future health conditions, a prediction model for myocardial infarction and the like are disclosed (Patent Literature 1).

### Citation List

### Patent Literature

Patent literature 1: JP 2022-184475A

### SUMMARY

### Technical Problem

Conventional prediction models as in Patent Literature 1 predict prognosis based on feature quantities. However, there is a further demand for encouraging individuals to proactively engage in one's health management.

An example object of the invention is to provide a program, a specific event related display apparatus, a specific event related display method, and a recording medium realizing display that can improve individual awareness of health management.

### Solution to Problem

To achieve the above object, a program for causing a computer to execute the following procedures, according to an example aspect of the present disclosure includes an information acquisition procedure, a display screen information generation procedure, and an information output procedure. The information acquisition procedure acquires future prediction information of a subject. The future prediction information of the subject includes future physical and mental state prediction information of the subject, and future living condition prediction information of the subject. The future physical and mental state prediction information of the subject includes information on occurrence risk of a specific event in future. The future living condition prediction information of the subject includes information on prediction of living condition associated with event occurrence in a case where the specific event occurs in the physical and mental state prediction information, and information on prediction of living condition associated with absence of event occurrence in a case where the specific event does not occur in the physical and mental state prediction information. The display screen information generation procedure generates a display screen including the information on occurrence risk of the specific event, the information on prediction of living conditions associated with event occurrence, and the information on prediction of living condition associated with absence of event occurrence, of the subject. The information output procedure outputs the display screen to a display device.

A specific event related display apparatus according to an example aspect of the present disclosure includes an information acquisition part, a display screen information generation part, and an information output part. The information acquisition part acquires future prediction information of a subject. The future prediction information of the subject includes future physical and mental state prediction information of the subject, and future living condition prediction information of the subject. The future physical and mental state prediction information of the subject includes information on occurrence risk of a specific event in future. The future living condition prediction information of the subject includes information on prediction of living condition associated with event occurrence in a case where the specific event occurs in the physical and mental state prediction information, and information on prediction of living condition associated with absence of event occurrence in a case where the specific event does not occur in the physical and mental state prediction information. The display screen information generation part generates a display screen including the information on occurrence risk of the specific event, the information on prediction of living condition associated with event occurrence, and the information on prediction of living condition associated with absence of event occurrence, of the subject. The information output part outputs the display screen to a display device.

A specific event related display method according to an example aspect of the present disclosure includes acquiring information, generating display screen information, and outputting information, which are executed by a computer. The acquiring information acquires future prediction information of a subject. The future prediction information of the subject includes future physical and mental state prediction information of the subject, and future living condition prediction information of the subject. The future physical and mental state prediction information of the subject includes information on occurrence risk of a specific event in future. The future living condition prediction information of the subject includes information on prediction of living condition associated with event occurrence in a case where the specific event occurs in the physical and mental state prediction information, and information on prediction of living condition associated with absence of event occurrence in a case where the specific event does not occur in the physical and mental state prediction information. The generating display screen information generates a display screen including the information on occurrence risk of the specific event, the information on prediction of living condition associated with event occurrence, and the information on prediction of living condition associated with absence of event occurrence, of the subject. The outputting information outputs the display screen to a display device.

A computer-readable recording medium recorded with a program for causing a computer to execute the following procedures, according to an example aspect of the present disclosure includes an information acquisition procedure, a display screen information generation procedure, and an information output procedure. The information acquisition procedure acquires future prediction information of a subject. The future prediction information of the subject includes future physical and mental state prediction information of the subject, and future living condition prediction information of the subject. The future physical and mental state prediction information of the subject includes information on occurrence risk of a specific event in future. The future living condition prediction information of the subject includes information on prediction of living condition associated with event occurrence in a case where the specific event occurs in the physical and mental state prediction information, and information on prediction of living condition associated with absence of event occurrence in a case where the specific event does not occur in the physical and mental state prediction information. The display screen information generation procedure generates a display screen including the information on occurrence risk of the specific event, the information on prediction of living condition associated with event occurrence, and the information on prediction of living condition associated with absence of event occurrence, of the subject. The information output procedure outputs the display screen to a display device.

### Advantageous Effects of Invention

An example advantage according to the present disclosure is that it becomes possible to improve individual awareness of health management by a displayed screen.

### BRIEF DESCRIPTION OF DRAWINGS

[FIG. 1] FIG. 1 is a flowchart showing an example procedure of the program according to the present disclosure.
[FIG. 2] FIG. 2 is a block diagram showing an example configuration of the specific event related display apparatus according to the present disclosure.
[FIG. 3] FIG. 3 is a block diagram showing an example hardware configuration of the specific event related display apparatus according to the present disclosure.
[FIG. 4] FIG. 4 is a reference diagram showing an example of a display screen in the program according to the present disclosure.
[FIG. 5] FIG. 5 is a reference diagram of example display of related information on a display screen in the program according to the present disclosure.
[FIG. 6] FIG. 6 is a reference diagram of an example display of another related information in the display screen in the program according to the present disclosure.
[FIG. 7] FIG. 7 is a reference diagram showing an example display of predicted values of future test results in the program according to the present disclosure.

### EXAMPLE EMBODIMENTS

The example embodiments of the present invention are described below. It is to be noted, however, that the present disclosure is by no means limited or restricted by the following example embodiments. In the following drawings, identical parts are indicated with identical reference signs. Regarding the descriptions of the example embodiments, reference can be made to one another unless otherwise stated. Furthermore, the configurations of the example embodiments can be combined unless otherwise stated. In each of the procedures in the program of the present disclosure described below, for example, "procedure" and "process" can be used interchangeably.

### First Example Embodiment

The program, the specific event related display apparatus, and the specific event related display method according to the present disclosure will be described. The program of the present disclosure is a program that causes a computer to execute each of the procedures described below. However, for example, the program may be used as an application on a user terminal, or may be used as a program on a server.

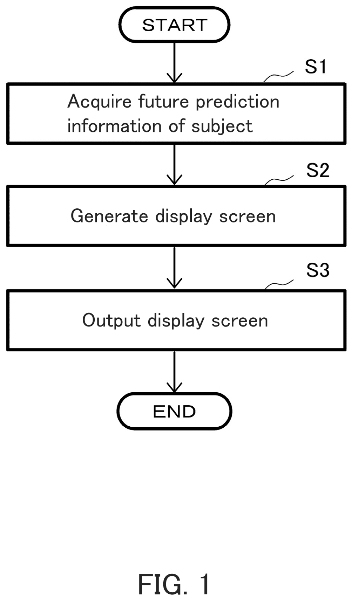

First, an example of the program of the present example embodiment will be described with reference to the flowchart of FIG. 1.

An information acquisition procedure acquires future prediction information of a subject (S1). The future prediction information of the subject includes future physical and mental state prediction information of the subject, and future living condition prediction information of the subject.

The future physical and mental state prediction information of the subject includes information on occurrence risk of a specific event in future. The specific event is, for example, development of a disease. The disease may include, for example, mental diseases. Further, the specific event may be, for example, improvement in physical strength, improvement in sleep, a sense of happiness, or the like, and is not limited to the one recognized as a disease, and includes a wide range of physical and mental states. The information on occurrence risk is not limited to likelihood of deterioration of the specific event of interest, and also includes, for example, likelihood of improvement, likelihood of maintaining the current state, and the like.

The future physical and mental state prediction information of the subject includes information on prediction of living condition associated with event occurrence, and information on prediction of living condition associated with absence of event occurrence. The information on prediction of living condition associated with event occurrence is information regarding prediction of living condition in a case where the specific event occurs in the physical and mental state prediction information. The information on prediction of living condition associated with absence of event occurrence is information regarding prediction of living condition in a case where the specific event does not occur in the physical and mental state prediction information. The living condition is, for example, economic burden, restriction of activities, necessity of hospitalization, or the like. The living condition is not limited thereto, and may include changes in physical condition, impacts on life, or the like, such as difficulty in reading text, and difficulty in hearing voices.

Specifically, the information on prediction of living condition associated with event occurrence is a simulation of living condition assuming the occurrence of the specific event, such as, specifically for example, if a disease develops, how much the hospitalization would cost, how long it would take to recover from the disease, and what kind of changes in physical condition, such as blurred vision, would occur. Note that, the content of the information on prediction of living condition associated with event occurrence is not limited to the examples described above. The information on prediction of living condition associated with absence of event occurrence is, for example, information that can be compared with the information on prediction of living condition associated with event occurrence, and is information regarding living condition that would be possible if the disease does not develop. The information on prediction of living condition associated with absence of event occurrence is, for example, a simulation of living condition assuming that the specific event does not occur, such as, for example, how many times a high-grade seafood hot pot can be ordered, how many times Kabuki play can be viewed in special seats, how many domestic World Heritage Sites can be visited, or the like, if there are available costs and time that would otherwise be necessary for disease treatment. Note that, the content of the information on prediction of living condition associated with absence of event occurrence is not limited to the examples described above, as in the case of the information on prediction of living condition associated with event occurrence. As described above, the information on prediction of future living condition of the subject is, for example, information allows comparison between the disadvantages in a case of disease development, and what specifically becomes possible in a case where there are no such disadvantages. Therefore, presentation of the information on the comparison motivates the subject to proactively engage in one's health management. In the above-described examples, deterioration of health condition is targeted. However, the specific event is not limited thereto. The specific event may be, for example, the one targeting recovery of health condition. In this case, for example, the information on the comparison is information that allows comparison between the disadvantages of not continuing the current lifestyle habits, and what specifically becomes possible when there are no such disadvantages. Presentation of the information on the comparison motivates the subject to continue one's previous lifestyle habits, and motivates the subject to proactively engage in one's health management.

A method for acquiring information in the information acquisition procedure may be, for example, acquiring information from an external device, such as information stored in an external database, information generated by an external device, or the like. Also, the generation of information to be acquired may be configured integrally with the program of the present disclosure. For example, the information acquired by the information acquisition procedure is stored in an external database in association with identification information of the subject. In this case, the information acquisition procedure, for example, acquires the identification information of the subject, and extracts and acquires the future prediction information of the subject that matches the identification information, from an external database. For example, in a case where an external device generates the future physical and mental state prediction information of the subject, the information acquisition procedure may be achieved by acquiring information used for the information generation such as test values, transmitting the acquired information to the external device, and receiving the information from the external device. For example, in a case where the future physical and mental state prediction information of the subject is generated in the program, the information acquisition procedure may be achieved by generating information used for the information generation such as test values, for the acquisition.

The display screen information generation procedure generates a display screen including the information on occurrence risk of the specific event, the information on prediction of living condition associated with event occurrence, and the information on prediction of living condition associated with absence of event occurrence, of the subject (S2). Display of these pieces of information in association with each other allows comparing and understanding these pieces of information. This makes it possible to improve individual awareness of health management.

The information output procedure outputs the display screen to the display device (S3). The display device displays the display screen on, for example, a dedicated monitor in a hospital or a personal computer used in an inspection institution, or the like. Note that, the information output procedure is not limited thereto. For example, the display screen may be printed to be outputted, outputted to an external database, or transmitted to a mobile terminal of the subject via an electric communication line to be displayed on the mobile terminal. In this way, the method of output by the information output procedure may adopt an appropriate method.

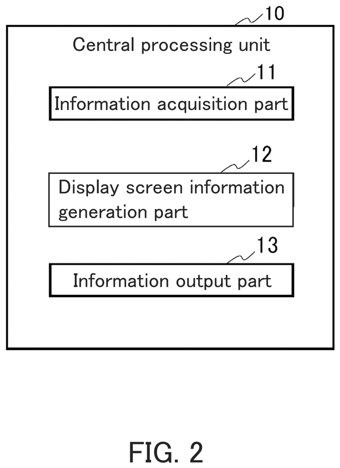

Next, with reference to FIG. 2, a specific event related display apparatus 10 (hereinafter, also referred to as "the apparatus") of the present example embodiment will be described. FIG. 2 is a block diagram showing an example configuration of the specific event related display apparatus of the present disclosure. As shown in FIG. 2, the apparatus includes an information acquisition part 11, a display screen information generation part 12, and an information output part 13. Although not shown, the apparatus may include, for example, an input unit, an output unit, a display unit, and/or a storage.

The apparatus may be, for example, one apparatus including the aforementioned parts, or may be an apparatus to which the respective parts are connectable via a communication line network. Further, the apparatus is connectable to an external device described later, via the communication line network. The communication line network is not particularly limited and may be a known network and may be, for example, wired or wireless. Examples of the communication line network include an internet line, WWW (World Wide Web), a telephone line, LAN (Local Area Network), SAN (Storage Area Network), DTN (Delay Tolerant Networking), LPWA (Low Power Wide Area), and L5G (local 5G). Examples of wireless communication include WI-FI^{®} (registered trademark), BLUETOOTH^{®} (registered trademark), local 5G, and LPWA. The wireless communication may be a form where apparatuses directly communicate with each other (Ad-Hoc communication), infrastructure communication, or may be indirect communication via an access point or the like. The apparatus may be built in a server as a system, for example. The apparatus may also be, for example, a personal computer (PC, e.g., a desktop PC and a laptop), a smartphone, a tablet terminal, or the like with the program of the present disclosure installed therein. The apparatus may be in a form of, for example, cloud computing, edge computing, or the like such that at least one of the aforementioned parts is on a server and the rest of the parts are on a terminal.

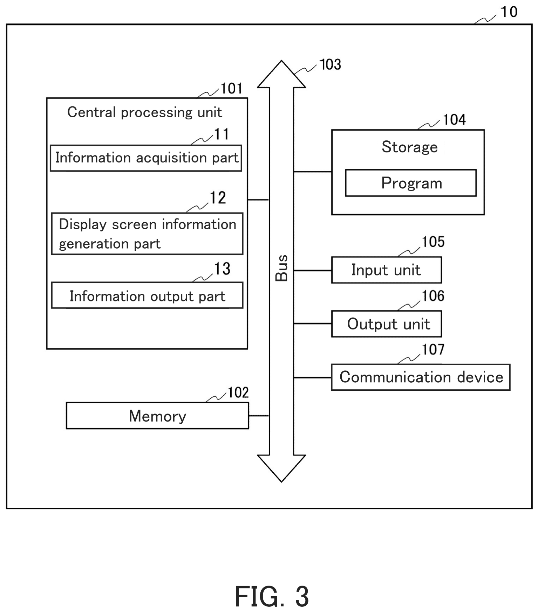

FIG. 3 is a block diagram showing an example hardware configuration of the apparatus. The apparatus includes, for example, a central processing unit (CPU, GPU, etc.) 101, a memory 102, a bus 103, a storage 104, an input unit 105, an output unit 106, a communication device 107, and the like. These components of the apparatus are connected to each other by their respective interfaces (I/F), via the bus 103.

The central processing unit 101 operates in cooperation with other configurations with a controller (a system controller, an I/O controller, or the like), and is responsible for control of the entire apparatus. In the apparatus, the central processing unit 101 executes, for example, the program of the present disclosure or other programs, and reads and writes various information. Specifically, for example, the central processing unit 101 functions as the information acquisition part 11, the display screen information generation part 12, and the information output part 13. The central processing unit 101 may include another arithmetic unit such as a CPU, a GPU (Graphics Processing Unit) and an APU (Accelerated Processing Unit), or a combination of these.

The bus 103 is connectable to an external device, for example. Examples of the external device include an external storage (e.g., external database), a printer, an external input unit, an external display device, and an external imaging device. The apparatus can be, for example, connected to an external network (the communication line network) through the communication device 107 connected to the bus 103, and can be connected to other devices via the external network.

The memory 102 may be, for example, a main memory (main storage). When the central processing unit 101 executes processing, for example, the memory 102 reads various operation programs such as the program of the present disclosure stored in the storage 104 to be described later, and the central processing unit 101 receives the data from the memory 102 to execute the program. The main memory is, for example, a RAM (Random-Access Memory). The memory 102 may be, for example, a ROM (Read-Only Memory).

The storage 104 is also referred to as a so-called auxiliary storage with respect to the main memory (main storage), for example. As mentioned above, the storage 104 stores operation programs including the program of the present disclosure. The storage 104 may be, for example, a combination of a recording medium and a drive for performing reading and writing on a recording medium. The recording medium is not particularly limited and may be a built-in type or an external type, and examples thereof include HD (Hard Disk), CD-ROM, CD-R, CD-RW, MO, DVD, a flash memory, and a memory card. The storage 104 may be, for example, a hard disk drive (HDD) or a solid state drive (SSD), in which a recording medium and a drive are integrated.

In the apparatus, the memory 102 and the storage 104 can also store various information such as log information, information acquired from an external data base (not shown) or an external device, information generated by the apparatus, and information used when the apparatus executes processing. In this case, the memory 102 and the storage 104 may store, for example, information or the like. At least a piece of the information may be, for example, stored in an external server other than the memory 102 and the storage 104, or may be dispersedly stored in a plurality of terminals using a blockchain technique or the like.

The apparatus further includes, for example, an input unit 105 and an output unit 106. Examples of the input unit 105 include a pointing device such as a touch panel, a track pad, and a mouse; a keyboard; an imaging device such as a camera and a scanner; a card reader such as an IC card reader and a magnetic card reader; and an audio inputting device such as a microphone. Examples of the output unit 106 include a display such as an LED display and a liquid crystal display; an audio output device such as a speaker; and a printer. In the first example embodiment, the input unit 105 and the output unit 106 are configured separately, but the input unit 105 and the output unit 106 may be configured integrally like a touch panel display.

Next, a method for processing information according to the present example embodiment will be described.

The specific event related display method of the present example embodiment can incorporate the description in the program of the present example embodiment. The specific event related display method of the present example embodiment is a method carried out with replacement of each "procedure" in the program of the present example embodiment with "process". Specifically, the specific event related display method of the present example embodiment includes processes of acquiring information, generating display screen information, and outputting information. The specific event related display method of the present example embodiment can be carried out using, for example, the apparatus of FIG. 2 or FIG. 3. Note that, the specific event related display method of the present example embodiment is not limited to the use of the apparatus of FIG. 2 or FIG. 3.

### Second Example Embodiment

Another example embodiment of the program, the specific event related display apparatus, and the specific event related display method according to the present disclosure will be described. In the present example embodiment, in which the specific event includes development of a specific disease, the procedures in the program, the configuration of the specific event related display apparatus, and the processes in the specific event related display method are the same as those in the first example embodiment, and thus the description thereof can be applied here.

First, an example of the program of the present example embodiment will be described. As described above, the information acquisition procedure (S1), the display screen information generation procedure (S2), and the information output procedure (S3) in the program of the present example embodiment are the same as those in the first example embodiment.

The future physical and mental state prediction information of the subject acquired in the information acquisition procedure includes, as described above, information on occurrence risk of a specific event in future. The specific event includes development of a specific disease. The specific disease includes, for example, diabetes, alcoholic liver disease, hepatic fibrosis, cirrhosis, hypertension, kidney disease, and the like, but is not limited to such physical diseases. The disease may include, for example, mental diseases. In addition, the information on occurrence risk is not limited to the risk of development of a specific disease, and may include, for example, likelihood of improvement of a specific disease, likelihood of maintaining the current condition, and the like.

The future living condition prediction information of the subject includes information on prediction of living condition associated with event occurrence, and information on prediction of living condition associated with absence of event occurrence. The information on prediction of living condition associated with event occurrence includes information on prediction of life associated with development of the specific disease. The information on prediction of life associated with development is information regarding the prediction of living condition in a case where the specific disease develops. The information on prediction of living condition associated with absence of event occurrence includes information on prediction of life associated with absence of development of the specific disease. The information on prediction of life associated with absence of development is information regarding the prediction of living condition in a case where the specific disease does not develop.

The information on life associated with development may include cost information, period information, and symptom information. The cost information is information on cost for dealing with the development of the specific disease, and is, for example, information on how much the hospitalization would cost if the specific disease develops. The cost information is not limited thereto, and may include, for example, costs to be spent in relation to the specific disease, such as outpatient cost, medication cost, transportation cost, and equipment cost. The period information is information on period for dealing with the development of the specific disease, and is, for example, information on how long the hospitalization would be if the specific disease develops. The period information is not limited thereto, and may widely include, for example, a period for complete cure, a period of being an outpatient, frequency of hospital visit, a period and degree of restraint for treatment of the specific disease, and the like. The symptom information is information on physical and mental symptoms in a case where the specific disease develops, and is, for example, information on what kind of changes in physical condition, such as blurred vision, would occur. The symptom information is not limited thereto, and may include a wide range of information regarding symptoms such as, for example, visual field defect, numbness in limbs, and the like. Therefore, the information on prediction of life associated with development of the specific disease is, for example, a simulation of living condition assuming the development of the specific disease, including cost information, period information, and symptom information. In contrast, the information on prediction of life associated with absence of development includes reward information. The reward information is information on rewards that the subject can obtain in a case where the specific disease does not develop. The reward information includes, for example, information on what would become possible if the cost associated with the specific disease is allocated to other costs. Therefore, the information on prediction of life associated with absence of occurrence includes information on, specifically for example, how many times a high-grade seafood hot pot can be ordered, how many times Kabuki play can be viewed in special seats, how many domestic World Heritage sites can be visited, and the like. That is, the information on prediction of life associated with absence of development is a simulation of living condition assuming that the specific disease does not develop. Note that, the information on prediction of life associated with development and the information on prediction of life associated with absence of development are not limited to the above-described examples.

As described above, the future living condition prediction information of the subject is, for example, information that allows comparison between the disadvantages in a case where a disease develops, and what specifically becomes possible in a case where there are no such disadvantages. Therefore, presentation of the information on the comparison motivates the subject to proactively engage in one's health management. In the above-described examples, deterioration of health condition is targeted, but the specific event is not limited thereto. The specific event may be, for example, the one targeting recovery of health condition. In this case, for example, when the displayed information indicates that the current lifestyle habits are effective, the information on the comparison be information that allows comparison between the disadvantages of not continuing the current lifestyle habits, and what specifically becomes possible in a case where there are no such disadvantages. Presentation of the information on the comparison encourages the subject to continue one's previous lifestyle habits, and motivates the subject to proactively engage in one's health management.

A method for acquiring information in the information acquisition procedure is the same as in the first example embodiment. The future prediction information of the subject is, for example, generated by an external device as described above. In this case, for example, a disease development risk is predicted based on the disease related information such as medical record information, health checkup information, and subject attribute information (sex, age, etc.), and the future physical and mental state prediction information of the subject among the future prediction information of the subject is generated based on the prediction. The disease development risk is predicted using, for example, a machine-learned prediction model. The disease development risk may also be predicted using a prediction device created on a rule base. The test values or the like used as information for the prediction may be predicted future test values. Further, the predicted test values may be used to predict the disease development risk. As described above, the future physical and mental state prediction information of the subject may be predicted, for example, using either artificial intelligence (AI) or a rule-based prediction device. The future living condition prediction information of the subject is, for example, stored in an external database in association with the content of the disease, but may be generated using a generation device that generates the information in accordance with the contents of test values, a disease development risk, or the like.

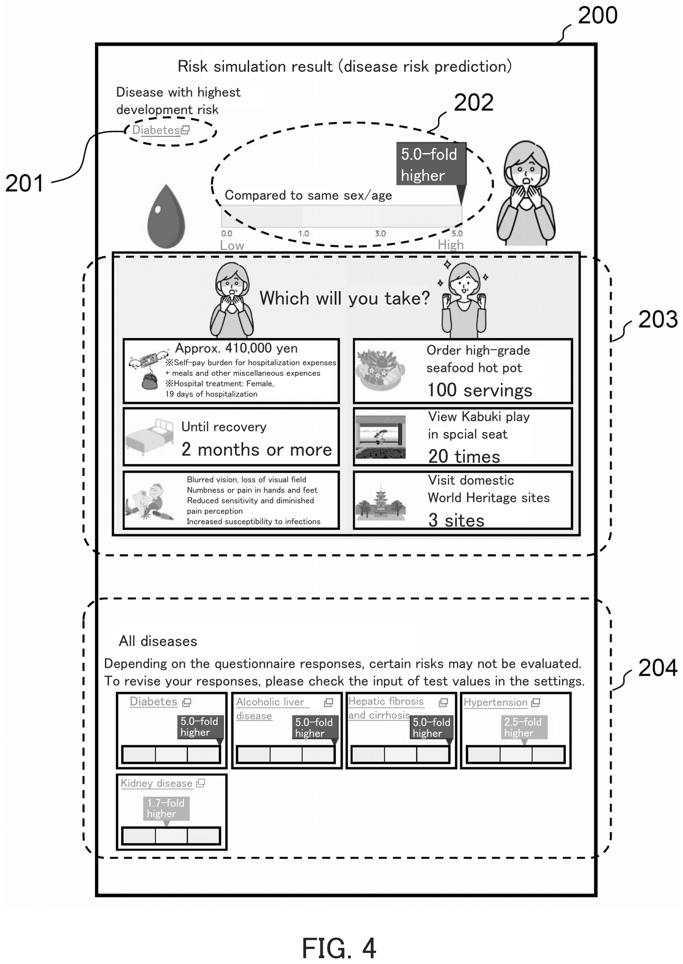

In the display screen information generation procedure, a display screen is generated as described in S2 above. FIG. 4 shows an example of the display screen in the program of the present disclosure. Note that, FIG. 4 is an example, and items, configuration, arrangement, and the like in the display screen are not limited thereto. In FIG. 4, a selected disease name 201 and a disease development risk 202 are displayed at the upper part of a display screen 200, and simulation 203 of the future living condition is arranged under the disease development risk 202. Further, under the simulation, summary information 204 on all diseases is displayed. First, in the summary information 204, the disease name and the disease development risk of each disease are displayed. When a disease name of interest is selected from the summary information 204, the disease name 201 and the disease development risk 202 regarding the selected disease are displayed in the upper part. Then, the simulation 203 arranged at the center is switched to the one corresponding to the selected disease. In the simulation 203, a simulation in case where the disease develops (information on prediction of living condition associated with event occurrence) is displayed on the left side, and a simulation in case a where the disease does not develop (information on prediction of living condition associated with absence of event occurrence) is displayed on the right side. By the above-described display, the subject can check at a glance a disease that may develop, a risk of development of the disease, and difference in living condition between the case where the disease develops and the case where the disease does not develop. By switching the selected disease in the summary information 204, it is possible to check the difference in living condition between the case where the disease of interest develops and the case where the disease does not develop, while comparing with the development risk. The display screen information generation procedure generates, for example, the above-described display screen, based on the future prediction information of the subject acquired in the information acquisition procedure.

The configuration of the specific event related display apparatus of the present example embodiment is the same as in the first example embodiment. Specifically, the specific event related display apparatus includes an information acquisition part 11, a display screen information generation part 12, and an information output unit 13. The processes in the specific event related display method of the present example embodiment are the same as in the first example embodiment. Specifically, the specific event related display method includes acquiring information, generating display screen information, and outputting information.

### Third Example Embodiment

Still another example embodiment of the program, the specific event related display apparatus, and the specific event related display method of the present disclosure will be described. In the present example embodiment, except that information to be acquired and information to be outputted differ, the procedures in the program, the configuration of the specific event related display apparatus, and the processes in the specific event related display method are the same as those in the first example embodiment, and thus the description thereof can be applied here.

First, an example of the program of the present example embodiment will be described. As described above, the information acquisition procedure (S1), the display screen information generation procedure (S2), and the information output procedure (S3) in the program of the present example embodiment are the same as in the first example embodiment. However, event related information is acquired in the information acquisition procedure (S1), and the display screen including the event related information is generated in the display screen information generation procedure (S2).

The event related information is information related to a specific event and is, for example, information on recommended tests. The event related information is not limited thereto, and may include information to be additionally displayed, such as lifestyle habits for health improvement, trivia information, and the like. A method for acquiring information is the same as in acquiring the future prediction information of the subject in other example embodiments. With regard to the event related information, the information may be acquired from an external database, the information generated by an external device may be acquired, or the program may be configured to generate the information. For example, when the event related information is related to a recommended test, the event related information may be generated in association with the prediction of the risk of development of the specific disease in the subject. In this way, the information acquisition procedure may acquire the event related information regarding the specific event.

The future prediction information of the subject acquired in the information acquisition procedure may include one or more pieces of future physical and mental test result prediction information of the subject. The event related information may include, for example, actual values such as test values or the like of the subject, or predicted values of test values may be acquired based on those actual values. Accordingly, the physical and mental test result prediction information is, for example, future test values predicted based on test values obtained in health checkups or the like of the subject. The physical and mental test result prediction information includes, for example, weight, blood pressure, cholesterol levels, and the like, but is not limited thereto, and may also include numerical values from mental tests, with no limitation on their content. The physical and mental test result prediction information may be the one expected when the lifestyle of the subject continues for a certain period. The physical and mental test result prediction information is not limited and may be, for example, prediction assuming that the current lifestyle habits continue for three years. The continuing period is not limited to three years and may be separately set, and prediction conditions may also be separately set.

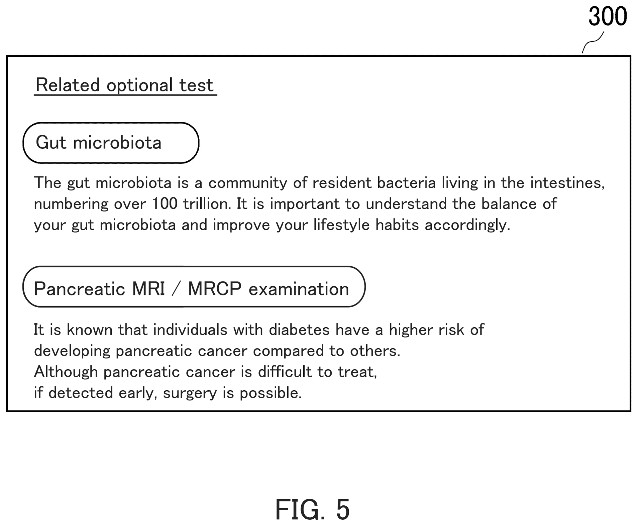

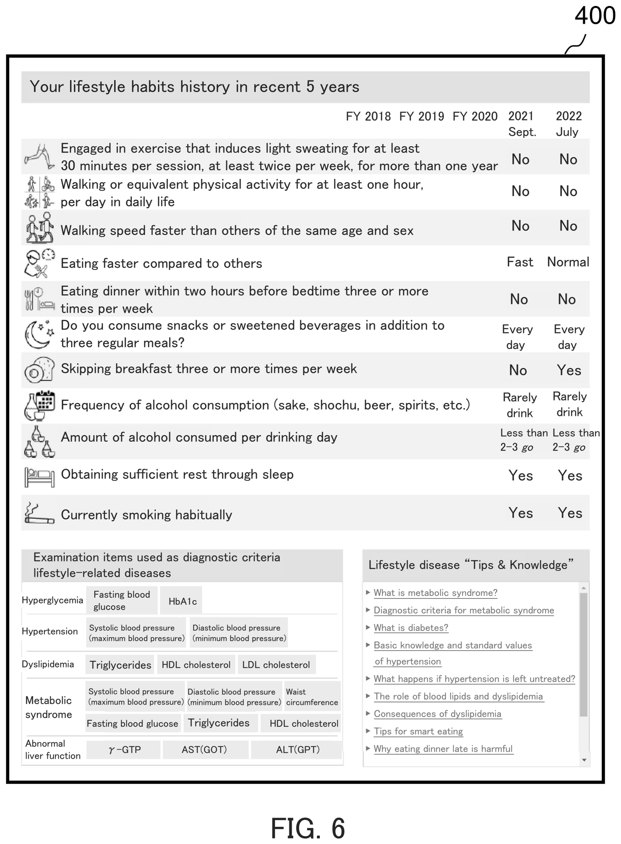

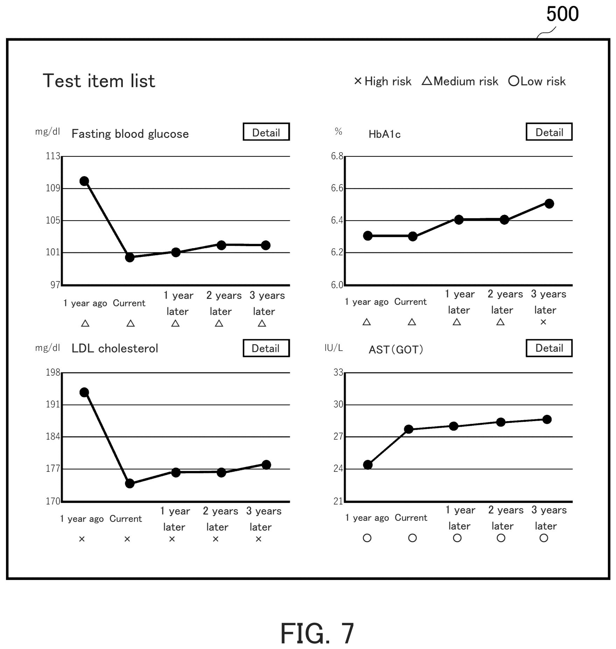

The display screen information generation procedure (S2) generates the display screen including the event related information. FIG. 5 and FIG. 6 are reference diagrams showing an example of display regarding the event related information. "Related optional test" in FIG. 5 is information on a test corresponding to the disease development risk. This display allows grasping what kind of test is appropriate, which can be utilized for health management. Note that, the presence of display, items, display method, arrangement, and the like for the recommended test may be freely set depending on actual circumstances. FIG. 6 shows display of lifestyle habits history. By checking this history, it is possible to grasp the relationship between the specific disease and lifestyle habits. In the same way, the display of lifestyle habits may also be freely set depending on actual circumstances. The event related information is not limited to the above-described examples, nor to information regarding a specific disease, and may be freely set depending on actual circumstances. FIG. 7 is an example of display of the physical and mental test result prediction information. In FIG. 7, a line graph is displayed. However, the display method is not limited thereto, and may be any method.

Next, the configuration of the specific event related display apparatus of the present example embodiment is the same as in the first example embodiment. Specifically, the specific event related display apparatus includes an information acquisition part 11, a display screen information generation part 12, and an information output unit 13. The processes in the specific event related display method of the present example embodiment are the same as in the first example embodiment. Specifically, the specific event related display method includes acquiring information, generating display screen information, and outputting information.

### Fourth Example Embodiment

The program of the present disclosure may be recorded in a computer-readable recording medium, for example. The recording medium is, for example, a non-transitory computer-readable recording medium (storage medium). The recording medium is not particularly limited and examples thereof include a random access memory (RAM), a read only memory (ROM), a hard disk (HD), a flash memory (e.g., a USB flash memory, and a SD/SDHC card), an optical disc (e.g., CD-R/CD-RW, DVD-R/DVD-RW, and BD-R/BD-RE), a magnetooptical disk (MO), and a FLOPPY^{®} (registered trademark) disk (FD). The program (may be referred to as a programming product or a program product, for example) of the present example embodiment may be in a form to be distributed from an external computer, for example. The "distribution" may be distribution via a communication line network or distribution via a wiredconnected device. The program according to the present example embodiment may be executed by being installed in a device where the program is distributed, or may be executed without being installed.

While the present disclosure has been particularly shown and described with reference to example embodiments thereof, the present disclosure is not limited to these example embodiments. It will be understood by those of ordinary skill in the art that various changes in form and details may be made therein without departing from the spirit and scope of the present disclosure. The example embodiments may be combined with each other as appropriate.

### SUPPLEMENTARY NOTE

The whole or part of the example embodiments disclosed above can be described as, but not limited to, the following supplementary notes.

### (Supplementary Note 1)

A program for causing a computer to execute the following procedures, including:
an information acquisition procedure;
a display screen information generation procedure, and
an information output procedure, wherein
the information acquisition procedure acquires future prediction information of a subject,
the future prediction information of the subject includes future physical and mental state prediction information of the subject, and future living condition prediction information of the subject,
the future physical and mental state prediction information of the subject includes information on occurrence risk of a specific event in future,
the future living condition prediction information of the subject includes information on prediction of living condition associated with event occurrence in a case where the specific event occurs in the physical and mental state prediction information, and information on prediction of living condition associated with absence of event occurrence in a case where the specific event does not occur in the physical and mental state prediction information,
the display screen information generation procedure generates a display screen including the information on occurrence risk of the specific event, the information on prediction of living condition associated with event occurrence, and the information on prediction of living condition associated with absence of event occurrence, of the subject, and
the information output procedure outputs the display screen to a display device.

### (Supplementary Note 2)

The program according to Supplementary Note 1, wherein
the specific event includes development of a specific disease,
the information on prediction of living condition associated with event occurrence includes information on prediction of life associated with development of the specific disease, and the information on prediction of living condition associated with absence of event occurrence includes information on prediction of life associated with absence of development of the specific disease.

### (Supplementary Note 3)

The program according to Supplementary Note 2, wherein
the information on prediction of life associated with development of the specific disease includes cost information, period information, and symptom information,
the cost information is information on cost for dealing with the specific disease,
the period information is information on period for dealing with the specific disease,
the symptom information is information on a physical or mental symptom in a case where the specific disease develops,
the information on prediction of life associated with absence of development of the specific disease includes reward information, and
the reward information is information on reward that the subject can obtain in a case where the specific disease does not develop.

### (Supplementary Note 4)

The program according to Supplementary Note 1, wherein
the information acquisition procedure acquires event related information regarding the specific event, and
the display screen information generation procedure generates the display screen including the event related information.

### (Supplementary Note 5)

The program according to Supplementary Note 1, wherein
the future prediction information of the subject includes one or more pieces of physical and mental test result prediction information of the subject, and
the display screen information generation procedure generates the display screen including the physical and mental test result prediction information.

### (Supplementary Note 6)

The program according to Supplementary Note 5, wherein
the physical and mental test result prediction information is the physical and mental test result prediction information predicted assuming that a lifestyle of the subject continues for a certain period.

### (Supplementary Note 7)

A specific event related display apparatus, including:
an information acquisition part,
a display screen information generation part, and
an information output part, wherein
the information acquisition part acquires future prediction information of a subject,
the future prediction information of the subject includes future physical and mental state prediction information of the subject, and future living condition prediction information of the subject,
the future physical and mental state prediction information of the subject includes information on occurrence risk of a specific event in future,
the future living condition prediction information of the subject includes information on prediction of living condition associated with event occurrence in a case where the specific event occurs in the physical and mental state prediction information, and information on prediction of living condition associated with absence of event occurrence in a case where the specific event does not occur in the physical and mental state prediction information,
the display screen information generation part generates a display screen including the information on occurrence risk of the specific event, the information on prediction of living condition associated with event occurrence, and the information on prediction of living condition associated with absence of event occurrence, of the subject, and
the information output part outputs the display screen to a display device.

### (Supplementary Note 8)

The apparatus according to Supplementary Note 7, wherein
the specific event includes development of a specific disease,
the information on prediction of living condition associated with event occurrence includes information on prediction of life associated with development of the specific disease, and the information on prediction of living condition associated with absence of event occurrence includes information on prediction of life associated with absence of development of the specific disease.

### (Supplementary Note 9)

The apparatus according to Supplementary Note 8, wherein
the information on prediction of life associated with development of the specific disease includes cost information, period information, and symptom information,
the cost information is information on cost for dealing with the specific disease,
the period information is information on period for dealing with the specific disease,
the symptom information is information on a physical or mental symptom in a case where the specific disease develops,
the information on prediction of life associated with absence of development of the specific disease includes reward information, and
the reward information is information on reward that the subject can obtain in a case where the specific disease does not develop.

### (Supplementary Note 10)

The apparatus according to Supplementary Note 7, wherein
the information acquisition part acquires event related information regarding the specific event, and
the display screen information generation part generates the display screen including the event related information.

### (Supplementary Note 11)

The apparatus according to Supplementary Note 7, wherein
the future prediction information of the subject includes one or more pieces of physical and mental test result prediction information of the subject, and
the display screen information generation part generates the display screen including the physical and mental test result prediction information.

### (Supplementary Note 12)

The apparatus according to Supplementary Note 11, wherein
the physical and mental test result prediction information is the physical and mental test result prediction information predicted assuming that a lifestyle of the subject continues for a certain period.

### (Supplementary Note 13)

A specific event related display method, including:
acquiring information;
generating display screen information, and
outputting information, wherein
the acquiring information acquires future prediction information of a subject,
the future prediction information of the subject includes future physical and mental state prediction information of the subject, and future living condition prediction information of the subject,
the future physical and mental state prediction information of the subject includes information on occurrence risk of a specific event in future,
the future living condition prediction information of the subject includes information on prediction of living condition associated with event occurrence in a case where the specific event occurs in the physical and mental state prediction information, and information on prediction of living condition associated with absence of event occurrence in a case where the specific event does not occur in the physical and mental state prediction information,
the generating display screen information generates a display screen including the information on occurrence risk of the specific event, the information on prediction of living condition associated with event occurrence, and the information on prediction of living condition associated with absence of event occurrence, of the subject, and
the outputting information outputs the display screen to a display device,
which are executed by a computer.

### (Supplementary Note 14)

The method according to Supplementary Note 13, wherein
the specific event includes development of a specific disease,
the information on prediction of living condition associated with event occurrence includes information on prediction of life associated with development of the specific disease, and
the information on prediction of living condition associated with absence of event occurrence includes information on prediction of life associated with absence of development of the specific disease.

### (Supplementary Note 15)

The method according to Supplementary Note 14, wherein
the information on prediction of life associated with development of the specific disease includes cost information, period information, and symptom information,
the cost information is information on cost for dealing with the specific disease,
the period information is information on period for dealing with the specific disease,
the symptom information is information on a physical or mental symptom in a case where the specific disease develops,
the information on prediction of life associated with absence of development of the specific disease includes reward information, and
the reward information is information on reward that the subject can obtain in a case where the specific disease does not develop.

### (Supplementary Note 16)

The method according to Supplementary Note 13, wherein
the acquiring information acquires event related information regarding the specific event, and the generating display screen information generates the display screen including the event related information.

### (Supplementary Note 17)

The method according to Supplementary Note 13, wherein
the future prediction information of the subject includes one or more pieces of physical and mental test result prediction information of the subject, and
the generating display screen information generates the display screen including the physical and mental test result prediction information.

### (Supplementary Note 18)

The method according to Supplementary Note 17, wherein
the physical and mental test result prediction information is the physical and mental test result prediction information predicted assuming that a lifestyle of the subject continues for a certain period.

### (Supplementary Note 19)

A computer-readable recording medium recorded with a program for causing a computer to execute the following procedures, including:
an information acquisition procedure;
a display screen information generation procedure, and
an information output procedure, wherein
the information acquisition procedure acquires future prediction information of a subject,
the future prediction information of the subject includes future physical and mental state prediction information of the subject, and future living condition prediction information of the subject,
the future physical and mental state prediction information of the subject includes information on occurrence risk of a specific event in future,
the future living condition prediction information of the subject includes information on prediction of living condition associated with event occurrence in a case where the specific event occurs in the physical and mental state prediction information, and information on prediction of living condition associated with absence of event occurrence in a case where the specific event does not occur in the physical and mental state prediction information,
the display screen information generation procedure generates a display screen including the information on occurrence risk of the specific event, the information on prediction of living condition associated with event occurrence, and the information on prediction of living condition associated with absence of event occurrence, of the subject.

### (Supplementary Note 20)

The recording medium according to Supplementary Note 19, wherein
the specific event includes development of a specific disease,
the information on prediction of living condition associated with event occurrence includes information on prediction of life associated with development of the specific disease, and the information on prediction of living condition associated with absence of event occurrence includes information on prediction of life associated with absence of development of the specific disease.

### (Supplementary Note 21)

The recording medium according to Supplementary Note 20, wherein
the information on prediction of life associated with development of the specific disease includes cost information, period information, and symptom information,
the cost information is information on cost for dealing with the specific disease,
the period information is information on period for dealing with the specific disease,
the symptom information is information on a physical or mental symptom in a case where the specific disease develops,
the information on prediction of life associated with absence of development of the specific disease includes reward information, and
the reward information is information on reward that the subject can obtain in a case where the specific disease does not develop.

### (Supplementary Note 22)

The recording medium according to Supplementary Note 19, wherein
the information acquisition procedure acquires event related information regarding the specific event, and
the display screen information generation procedure generates the display screen including the event related information.

### (Supplementary Note 23)

The recording medium according to Supplementary Note 19, wherein
the future prediction information of the subject includes one or more pieces of physical and mental test result prediction information of the subject, and
the display screen information generation procedure generates the display screen including the physical and mental test result prediction information.

### (Supplementary Note 24)

The recording medium according to Supplementary Note 23, wherein
the physical and mental test result prediction information is the physical and mental test result prediction information predicted assuming that a lifestyle of the subject continues for a certain period.

### Industrial Applicability

The present invention can realize, for example, obtaining disease prediction information, recommended test information, and information on statistical analysis result for each of a plurality of body sites of a disease prediction subject, from information related to a disease obtained through health checkup or the like. Thus, the disease prediction subject can comprehensively grasp one's health condition, and the present invention is useful for appropriate health management of the disease prediction subject.

### Reference Signs List

- 10:: specific event related display apparatus
- 11:: information acquisition part
- 12:: display screen information generation part
- 13:: information output part
- 101:: central processing unit
- 102:: memory
- 103:: bus
- 104:: storage
- 105:: input unit
- 106:: output unit
- 107:: communication device

## Claims

1. A program for causing a computer to execute the following procedures, comprising:
an information acquisition procedure;
a display screen information generation procedure, and
an information output procedure, wherein
the information acquisition procedure acquires future prediction information of a subject,
the future prediction information of the subject includes future physical and mental state prediction information of the subject, and future living condition prediction information of the subject,
the future physical and mental state prediction information of the subject includes information on occurrence risk of a specific event in future,
the future living condition prediction information of the subject includes information on prediction of living condition associated with event occurrence in a case where the specific event occurs in the physical and mental state prediction information, and information on prediction of living condition associated with absence of event occurrence in a case where the specific event does not occur in the physical and mental state prediction information,
the display screen information generation procedure generates a display screen including the information on occurrence risk of the specific event, the information on prediction of living condition associated with event occurrence, and the information on prediction of living condition associated with absence of event occurrence, of the subject, and
the information output procedure outputs the display screen to a display device.

2. The program according to claim 1, wherein
the specific event includes development of a specific disease,
the information on prediction of living condition associated with event occurrence includes information on prediction of life associated with development of the specific disease, and
the information on prediction of living condition associated with absence of event occurrence includes information on prediction of life associated with absence of development of the specific disease.

3. The program according to claim 2, wherein
the information on prediction of life associated with development of the specific disease includes cost information, period information, and symptom information,
the cost information is information on cost for dealing with the specific disease,
the period information is information on period for dealing with the specific disease,
the symptom information is information on a physical or mental symptom in a case where the specific disease develops,
the information on prediction of life associated with absence of development of the specific disease includes reward information, and
the reward information is information on reward that the subject can obtain in a case where the specific disease does not develop.

4. The program according to claim 1, wherein
the information acquisition procedure acquires event related information regarding the specific event, and
the display screen information generation procedure generates the display screen including the event related information.

5. The program according to claim 1, wherein
the future prediction information of the subject includes one or more pieces of physical and mental test result prediction information of the subject, and
the display screen information generation procedure generates the display screen including the physical and mental test result prediction information.

6. The program according to claim 5, wherein
the physical and mental test result prediction information is the physical and mental test result prediction information predicted assuming that a lifestyle of the subject continues for a certain period.

7. A specific event related display apparatus, comprising:
an information acquisition part,
a display screen information generation part, and
an information output part, wherein
the information acquisition part acquires future prediction information of a subject,
the future prediction information of the subject includes future physical and mental state prediction information of the subject, and future living condition prediction information of the subject,
the future physical and mental state prediction information of the subject includes information on occurrence risk of a specific event in future,
the future living condition prediction information of the subject includes information on prediction of living condition associated with event occurrence in a case where the specific event occurs in the physical and mental state prediction information, and information on prediction of living condition associated with absence of event occurrence in a case where the specific event does not occur in the physical and mental state prediction information,
the display screen information generation part generates a display screen including the information on occurrence risk of the specific event, the information on prediction of living condition associated with event occurrence, and the information on prediction of living condition associated with absence of event occurrence, of the subject, and
the information output part outputs the display screen to a display device.

8. The apparatus according to claim 7, wherein
the specific event includes development of a specific disease,
the information on prediction of living condition associated with event occurrence includes information on prediction of life associated with development of the specific disease, and
the information on prediction of living condition associated with absence of event occurrence includes information on prediction of life associated with absence of development of the specific disease.

9. The apparatus according to claim 8, wherein
the information on prediction of life associated with development of the specific disease includes cost information, period information, and symptom information,
the cost information is information on cost for dealing with the specific disease,
the period information is information on period for dealing with the specific disease,
the symptom information is information on a physical or mental symptom in a case where the specific disease develops,
the information on prediction of life associated with absence of development of the specific disease includes reward information, and
the reward information is information on reward that the subject can obtain in a case where the specific disease does not develop.

10. The apparatus according to claim 7, wherein
the information acquisition part acquires event related information regarding the specific event, and
the display screen information generation part generates the display screen including the event related information.

11. The apparatus according to claim 7, wherein
the future prediction information of the subject includes one or more pieces of physical and mental test result prediction information of the subject, and
the display screen information generation part generates the display screen including the physical and mental test result prediction information.

12. The apparatus according to claim 11, wherein
the physical and mental test result prediction information is the physical and mental test result prediction information predicted assuming that a lifestyle of the subject continues for a certain period.

13. A specific event related display method, comprising:
acquiring information;
generating display screen information, and
outputting information, wherein
the acquiring information acquires future prediction information of a subject,
the future prediction information of the subject includes future physical and mental state prediction information of the subject, and future living condition prediction information of the subject,
the future physical and mental state prediction information of the subject includes information on occurrence risk of a specific event in future,
the future living condition prediction information of the subject includes information on prediction of living condition associated with event occurrence in a case where the specific event occurs in the physical and mental state prediction information, and information on prediction of living condition associated with absence of event occurrence in a case where the specific event does not occur in the physical and mental state prediction information,
the generating display screen information generates a display screen including the information on occurrence risk of the specific event, the information on prediction of living condition associated with event occurrence, and the information on prediction of living condition associated with absence of event occurrence, of the subject, and
the outputting information outputs the display screen to a display device,
which are executed by a computer.

14. The method according to claim 13, wherein
the specific event includes development of a specific disease,
the information on prediction of living condition associated with event occurrence includes information on prediction of life associated with development of the specific disease, and
the information on prediction of living condition associated with absence of event occurrence includes information on prediction of life associated with absence of development of the specific disease.

15. The method according to claim 14, wherein
the information on prediction of life associated with development of the specific disease includes cost information, period information, and symptom information,
the cost information is information on cost for dealing with the specific disease,
the period information is information on period for dealing with the specific disease,
the symptom information is information on a physical or mental symptom in a case where the specific disease develops,
the information on prediction of life associated with absence of development of the specific disease includes reward information, and
the reward information is information on reward that the subject can obtain in a case where the specific disease does not develop.

16. The method according to claim 13, wherein
the acquiring information acquires event related information regarding the specific event, and
the generating display screen information generates the display screen including the event related information.

17. The method according to claim 13, wherein
the future prediction information of the subject includes one or more pieces of physical and mental test result prediction information of the subject, and
the generating display screen information generates the display screen including the physical and mental test result prediction information.

18. The method according to claim 17, wherein
the physical and mental test result prediction information is the physical and mental test result prediction information predicted assuming that a lifestyle of the subject continues for a certain period.

19. A computer-readable recording medium recorded with a program for causing a computer to execute the following procedures, comprising:
an information acquisition procedure;
a display screen information generation procedure, and
an information output procedure, wherein
the information acquisition procedure acquires future prediction information of a subject,
the future prediction information of the subject includes future physical and mental state prediction information of the subject, and future living condition prediction information of the subject,
the future physical and mental state prediction information of the subject includes information on occurrence risk of a specific event in future,
the future living condition prediction information of the subject includes information on prediction of living condition associated with event occurrence in a case where the specific event occurs in the physical and mental state prediction information, and information on prediction of living condition associated with absence of event occurrence in a case where the specific event does not occur in the physical and mental state prediction information,
the display screen information generation procedure generates a display screen including the information on occurrence risk of the specific event, the information on prediction of living condition associated with event occurrence, and the information on prediction of living condition associated with absence of event occurrence, of the subject.

20. The recording medium according to claim 19, wherein
the specific event includes development of a specific disease,
the information on prediction of living condition associated with event occurrence includes information on prediction of life associated with development of the specific disease, and
the information on prediction of living condition associated with absence of event occurrence includes information on prediction of life associated with absence of development of the specific disease.
